(19) Europäisches Patentamt
European Patent Office
Office européen des brevets

(11) Veröffentlichungsnummer: **0 247 970**
**B1**

(12) # EUROPÄISCHE PATENTSCHRIFT

(45) Veröffentlichungstag der Patentschrift: **04.07.90**

(51) Int. Cl.⁵: **C07D 307/77**, C07C 255/56, C07C 59/84, C09B 29/36

(21) Anmeldenummer: **87810303.5**

(22) Anmeldetag: **20.05.87**

(54) **Benzanthronlactone, Verfahren zu ihrer Herstellung und ihre Verwendung.**

(30) Priorität: **26.05.86 CH 2113/86**

(43) Veröffentlichungstag der Anmeldung: **02.12.87 Patentblatt 87/49**

(45) Bekanntmachung des Hinweises auf die Patenterteilung: **04.07.90 Patentblatt 90/27**

(84) Benannte Vertragsstaaten: **BE CH DE FR GB IT LI**

(56) Entgegenhaltungen: **FR-A- 754 842**

(73) Patentinhaber: **CIBA-GEIGY AG, Klybeckstrasse 141, CH-4002 Basel(CH)**

(72) Erfinder: **Weis, Claus D., Dr., Hangelimattweg 112, CH-4148 Pfeffingen(CH)**
Erfinder: **Sutter, Peter, Seemättlistrasse 14/2, CH-4132 Muttenz(CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 247 970 B1

## Beschreibung

Die vorliegende Erfindung betrifft Benzanthronlactone, Verfahren zu deren Herstellung sowie neue Farbstoffe, die diese als Kupplungskomponente enthalten.

Benzanthron und eine Vielzahl substituierter Derivate sind z.B aus der FR-A 754 842 seit langem z.B. als Küpenfarbstoffe sowie als Zwischenprodukte für die Herstellung von Farbstoffen und Pigmente bekannt, jedoch wurden bis jetzt noch keine Benzanthronlactone beschrieben.

Es wurden nun neue Benzanthronlactone gefunden, welche überraschende Eigenschaften haben und ausserdem wichtige Zwischenprodukte z.B. für die Synthese von Farbstoffen darstellen.

Gegenstand der vorliegenden Erfindung sind demgemäss Benzanthronlactone der Formel

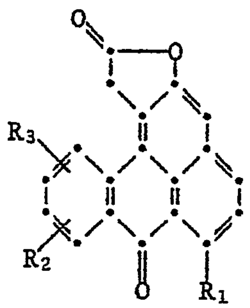

(1) ,

worin $R_1$ Wasserstoff oder Hydroxy und $R_2$ und $R_3$ unabhängig voneinander Wasserstoff, Chlor, Hydroxy, Methoxy oder Benzamido bedeuten.

$R_1$ steht hierbei bevorzugt für Wasserstoff.

$R_2$ und $R_3$ haben unabhängig voneinander bevorzugt die Bedeutung Wasserstoff, Chlor oder Benzamido und stehen besonders bevorzugt für Wasserstoff.

In einer bevorzugten Ausführungsform der Benzanthronlactone haben $R_1$, $R_2$ und $R_3$ jeweils die Bedeutung Wasserstoff.

Die erfindungsgemässen Benzanthronlactone lassen sich herstellen, indem man ein Anthrachinon-1-diazoniumsalz der Formel

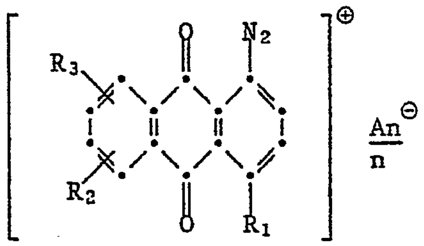

(2) ,

worin $R_1$, $R_2$ und $R_3$ die im Anspruch 1 angegebene Bedeutung haben, An ein Anion ist und n dessen Wertigkeit angibt, mit 2-Methylenglutarsäuredinitril in Gegenwart von katalytischen Mengen eines Metalls der VIII. oder I. Nebengruppe, einem Salz dieser Metalle oder einer Mischung aus einem entsprechenden Metallsalz und Metallpulver in einem organischen Lösungsmittel umsetzt, das hierbei erhältliche Zwischenprodukt der Formel

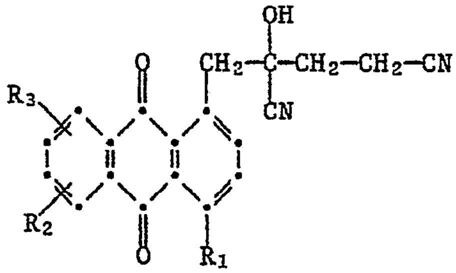

(3)

zum Produkt der Formel

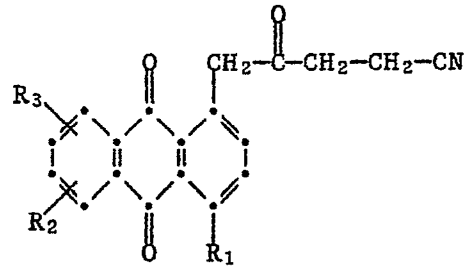

(4)

reagieren lässt und dieses in alkalischem Medium zum Benzanthronlacton der Formel (1) cyclisiert.

Bei den Anion An handelt es sich z.B. um ein Chlorid- oder Sulfatanion und n hat dementsprechend z.B. die Bedeutung 1 oder 2.

Das Anthrachinon-1-diazoniumsalz ist vorzugsweise ein Anthrachinon-1-diazoniumsulfat.

Die Anthrachinon-1-diazoniumsalze werden bevorzugt in festem Zustand, und zwar in Form eines trockenen Pulvers oder einer wasserfeuchten Paste verwendet. Sie können durch Diazotierung der entsprechenden 1-Aminoanthrachinone in konzentrierter Schwefelsäure und durch nachfolgende Ausfällung mit Wasser und Eis in an sich bekannter Weise erhalten werden. Das derart ausgefällte Diazoniumsalz kann nach Abfiltrieren und Waschen mit Wasser entweder unter Vakuum bei niederer Temperatur getrocknet oder direkt in noch feuchtem Zustand verwendet werden.

Das 2-Methylenglutarsäuredinitril wird in stöchiometrischen Mengen oder vorzugsweise im Ueberschuss, bezogen auf das Anthrachinon-1-diazoniumsalz eingesetzt; besonders vorteilhaft ist hierbei die Verwendung eines ein- bis zehnfachen und insbesondere bevorzugt die eines zwei- bis sechsfachen molaren Ueberschusses an 2-Methylenglutarsäuredinitril, bezogen auf das Diazoniumsalz.

Als Katalysator für die Umsetzung des Anthrachinon-1-diazoniumsalzes mit 2-Methylenglutarsäuredinitril kommen z.B. Eisen- oder Kupferpulver, Eisen- oder Kupfersalze oder aus Eisenpulver und -salz oder Kupferpulver und -salz bestehende Gemische in Frage; bei den Metallsalzen handelt es sich bevorzugt um die entsprechenden Chloride. Wird ein Gemisch aus Metallpulver und -salz verwendet, so werden beide bevorzugt im Verhältnis 1:1 eingesetzt.

Vorzugsweise benutzt man katalytische Mengen Kupferpulver, Kupfer-(I)- oder Kupfer-(II)-chlorid und insbesondere bevorzugt ist die Verwendung von Kupfer-(I)-chlorid.

Der Katalysator wird in Mengen von z.B. 1 bis 5 Gew.-%, bezogen auf das Anthrachinon-1-diazoniumsalz, eingesetzt.

Als Lösungsmittel für die Umsetzung des Anthachinon-1-diazoniumsalzes mit 2-Methylenglutarsäuredinitril kommen insbesondere polare Lösungsmittel, z.B. $C_1$-$C_4$-Alkanole wie Methanol, Ethanol, n- oder iso-Propanol oder n- oder iso-Butanol, Nitrile wie Acetonitril oder Isopentylnitril, Ketone wie Aceton oder phosphorhaltige Verbindungen wie z.B. Methanphosphonsäuredimethylester in Frage; bevorzugt ist die Verwendung von $C_1$-$C_4$-Alkanolen oder phosphorhaltigen Verbindungen und besonders bevorzugt diejenige von Methanphosphonsäuredimethylester oder insbesondere von Methanol.

Die Umsetzung des Anthrachinon-1-diazoniumsalzes mit 2-Methylenglutarsäuredinitril wird zweckmässigerweise bei einer Temperatur von 20 bis 100°C ausgeführt.

Das Cyanhydrin der Formel (3) lässt sich bei einer Temperatur von ca. 40 bis 180°C und vorzugsweise von 70 bis 150°C in das Ketonitril der Formel (4) überführen. Ein für diese Umsetzung brauchbares Medium besteht aus einem inerten Lösungsmittel und einem sauren Ionenaustauscher. Als inerte Lösungsmittel kommen hierbei z.B. Alkanole wie Ethanol, n- oder iso-Propanol oder n- oder iso-Butanol oder vorzugsweise aromatische Kohlenwasserstoffe wie Toluol, Xylol oder Chlorbenzol in Frage. Geeignete Ionenaustauscher sind z.B. stark saure kationische Ionenaustauscher und hierbei insbesondere solche auf Kunstharz-Basis wie z.B. Ionenaustauscher mit Sulfonsäuregruppen.

Man benutzt für die Cyclisierung ein alkalisches Medium, welches z.B. aus einer Base und einem Lösungsmittel besteht. Als Basen kommen z.B. Alkalimetallhydroxide wie Natrium- oder Kaliumhydroxid, Alkoholate wie Natrium- oder Kaliummethanolat oder -ethanolat oder Alkalimetallcarbonate wie Natrium- oder Kaliumcarbonat in Frage.

Geeignete Lösungsmittel sind z.B. polare aprotische Lösungsmittel wie Dimethylformamid, Dimethylsulfoxid, Hexamethylphosphorsäuretriamid oder Glycolether, z.B. Diethylenglycoldiethylether oder Diethylenglycoldimethylether.

Die Cyclisierung wird bei einer Temperatur von ≤ 80°C, vorzugsweise bei 40 bis 70°C, durchgeführt. Überraschenderweise erfolgt dabei nicht nur die Cyclisierung des Ketonitrils (4) zum Benzanthron, sondern auch ein zweiter Ringschluss zum Lacton.

Ein weiterer Gegenstand der Erfindung betrifft Cyanhydrine der zuvor angegebenen Formel (3), wobei für $R_1$, $R_2$ und $R_3$ die zuvor angegebenen Bedeutungen und Bevorzugungen gelten.

Die Erfindung betrifft ausserdem Verbindungen der Formel

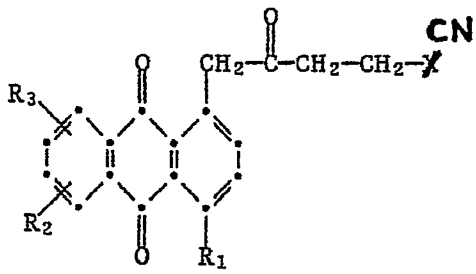

(4) ,

wobei für $R_1$, $R_2$ und $R_3$ die zuvor angegebenen Bedeutungen und Bevorzugungen gelten.

Die Verbindungen der Formeln (3) und (4) sind wertvolle Synthesebausteine, von denen ausgehend sich eine grosse Zahl substituierter Anthrachinone sowie Heterocyclen synthetisieren lässt.

Die erfindungsgemässen Benzanthronlactone der Formel (1) ergeben mit primären, sekundären oder tertiären Aminen, insbesondere mit hochsiedenden Aminen mit einem Siedepunkt von ≥150°C, z.B. Dibutylamin, Diisobutylamin, Tripropylamin, Tetramethylbutylamin, N,N-Dimethyl- oder N,N-Diethylanilin, tiefblaue Färbungen.

Die erfindungsgemässen Benzanthronlactone stellen darüberhinaus wertvolle Kupplungskomponenten für die Synthese von neuen Hydrazono-Farbstoffen dar.

Ein weiterer Gegenstand der Erfindung sind daher Farbstoffe der Formel

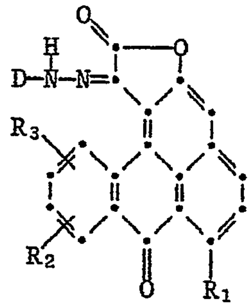

(5)

und tautomere Formen davon, wobei $R_1$, $R_2$ und $R_3$ die zuvor angegebene Bedeutung haben und D der Rest einer Diazokomponente ist.

Hierbei gelten für $R_1$, $R_2$ und $R_3$ die zuvor angegebenen Bevorzugungen.

Der Diazorest D leitet sich z.B. von einem aromatischen carbocyclischen oder heterocyclischen Amin der allgemeinen Formel

$D\text{-}NH_2$

ab. Vorzugsweise handelt es sich bei dem Amin um ein Aminobenzol oder um eine mono- oder bicyclische Verbindung, die einen aromatisch-heterocyclischen 5- oder 6-Ring aufweist.

Das Aminobenzol und das heterocyclische Amin können noch einen oder mehrere weitere Substituenten tragen, z.B. $C_1$-$C_4$-Alkyl wie Methyl, Ethyl, n- oder iso-Propyl oder n-, sec.- oder tert.-Butyl, $C_1$-$C_4$-Alkoxy und hierbei insbesondere Methoxy, Halogen wie Brom und insbesondere Chlor, Nitro, Cyano, Sulfo, Carboalkoxy wie Carbomethoxy oder Carboethoxy, Alkylsulfonyl wie Methylsulfonyl oder unsubstituiertes, mono- oder dialkyliertes oder aryliertes Sulfonamid wie N-Methyl-, N-Phenyl- oder N,N-Dimethylsulfonamid.

Handelt es sich bei dem Amin um ein heterocyclisches Amin, so kann der zugrunde liegende Heterocyclus z.B. ein Thiazol, Benzthiazol, Benzisothiazol, Pyridin, Chinolin, Pyrazol, Thiophen oder Phthalimid sein.

Beispiele für geeignete heterocyclische Amine sind:

2-Aminothiazol, 2-Amino-5-nitrothiazol, 3-Amino-benzisothiazol, 3-Aminopyridin, 3-Aminochinolin, 2-Amino-6-methylbenzthiazol, 2-Amino-4-cyanopyrazol, 3- oder 4-Aminophthalimid.

Der Diazorest D leitet sich bevorzugt von einem Aminobenzol ab; das Aminobenzol ist hierbei besonders bevorzugt unsubstituiert oder mit Methoxy, Chlor, Nitro, Cyano und/oder Methyl weitersubstituiert.

Beispiele für geeignete Aminobenzole sind:

Anilin, 1-Amino-4-chlorbenzol, 1-Amino-2-cyano-4-chlorbenzol, 1-Amino-2-carbomethoxy-4-chlorbenzol, 4-Nitroanilin, Sulfanilsäure, 4-Methoxyanilin, 3,5-Dinitroanilin, 1-Aminobenzol-2-, -3- oder 4-sulfonsäureamid, 1-Amino-4-methylsulfonylbenzol, 1-Amino-2,4-dicyanobenzol, 1-Amino-2-cyano-4-nitrobenzol, 4-Chlor-2-nitroanilin.

Die neuen Hydrazonofarbstoffe der Formel (5) lassen sich in an sich bekannter Weise herstellen, z.B., indem man eine Diazokomponente der Formel

$D\text{-}NH_2$

diazotiert und auf ein Benzanthronlacton der Formel (1) kuppelt.

Die neuen Verbindungen oder deren Mischungen miteinander sind für das Färben und Bedrucken z.B. von Leder, Wolle, Seide und von synthetischen Fasern, wie beispielsweise von Acryl- oder Acrylnitrilfasern, Polyacrylnitrilfasern und Copolymerisaten von Acrylnitril und anderen Vinylverbindungen, wie beispielsweise Acrylester, Acrylamide, Vinylpyridin, Vinylchlorid oder Vinylidenchlorid, Copolymerisaten von Dicyanoethylen und Vinylacetat, und von Acrylnitril-Blockcopolymerisaten, Fasern von Polyurethan, Polyolefinen, wie beispielsweise basisch modifiziertes Polypropylen, Polypropylen modifiziert mit Nickel oder unmodifiziertes Polypropylen, Cellulosetriacetat und Cellulose-2 1/2-acetat, und Fasern von Polyamiden, wie beispielsweise Nylon-6, Nylon-6,6 oder Nylon-12, und von aromatischen Polyestern, wie beispielsweise denjenigen aus Terephthalsäure und Ethylenglykol, geeignet.

Ein bevorzugtes Einsatzgebiet der neuen Farbstoffe der Formel (5), welche keine wasserlöslichmachenden Gruppen aufweisen, betrifft ihre Verwendung zum Färben von synthetischen faserbildenden Polymerisaten wie Polyamiden, Polyolefinen und insbesondere Polyestern. Die Fasern können hierbei z.B. gemäss den herkömmlichen Verfahren für Polyestermaterialien gefärbt werden. Es werden kräftige in der Regel rot bis orange farbene Ausfärbungen oder auch Drucke mit guten Echtheitseigenschaften erhalten, insbesondere einer guten Echtheit gegenüber Thermofixieren, Sublimation, Plissieren, Abgas, Ueberfärben, Trockenreinigen und Chlor, und gute Nassechtheitseigenschaften, beispielsweise Echtheit gegenüber Wasser, Waschen und Schweiss.

In den nachfolgenden Beispielen bedeuten Teile Gewichtsteile und die Temperaturen sind in Grad Celsius angegeben.

<u>Beispiel 1:</u> 250 Teile 1-Aminoanthrachinon werden bei einer Temperatur von 50°C innerhalb von 1 Stunde zu einer Lösung von 85 Teilen Natriumnitrit in 750 ml konzentrierter $H_2SO_4$ dosiert und anschliessend eine halbe Stunde bei dieser Temperatur weitergerührt. Das Reaktionsgemisch wird dann auf Eis gegeben, das resultierende Diazoniumsalz abgesaugt und mit Eiswasser gewaschen.

Das durch Ausdrücken so weit wie möglich getrocknete Diazioniumsalz wird in einem Gemisch aus 2000 ml Methanol und 530 Teilen 2-Methylenglutarsäuredinitril aufgeschlämmt, und die resultierende Suspension auf 40°C erhitzt. Dann werden 4 Teile Kupfer-(I)-chlorid innerhalb von 15 Minuten in kleinen Portionen hinzugegeben. Nach dem Abklingen der $N_2$-Entwicklung wird die Temperatur auf 50 bis 55°C erhöht und 1,5 Stunden bei dieser Temperatur gehalten. Danach kühlt man die Reaktionslösung auf etwa 10°C ab und filtriert 37,5 Teile Anthrachinon ab. Die verbleibende Lösung wird eingeengt und 282 Teile Rohprodukt durch Filtration gewonnen, welche anschliessend noch in einem Soxhlett mit Cyclohexan extrahiert werden. Es ergeben sich 267 Teile 3-Hydroxy-4-(anthrachinon-1-yl)-butan-1,3-dinitril. Schmelzpunkt 144-146°C.

<u>Beispiel 2:</u> 8 Teile 3-Hydroxy-4-(anthrachinon-1-yl)-butan-1,3-dinitril gemäss Beispiel 1 werden zusammen mit 0,8 Teilen neutralem Aluminiumoxid in 50 ml Xylol suspendiert und 40 Minuten unter Rückfluss erhitzt. Die Reaktionslösung wird nach Zugabe von Aktiv kohle heiss filtriert und das Filtrat zur Trockene eingedampft. Nach dem Umkristallisieren ergeben sich 5,9 Teile 3-Oxo-4-(anthrachinon-1-yl)-butyronitril. Schmelzpunkt 165-167°C.

<u>Beispiel 3:</u> 5 Teile 3-Oxo-4-(anthrachinon-1-yl)-butyronitril gemäss Beispiel 2 werden 2 Stunden in 40 ml konzentrierter Schwefelsäure gerührt; danach wird die Reaktionslösung langsam in 200 Teile Eiswasser gegeben und weitere 12 Stunden gerührt. Der Feststoff wird abgesaugt und gewaschen. Ausbeute 5,1 Teile 3-Oxo-4-(anthrachinon-1-yl)-buttersäure; Schmelzpunkt nach Umkristallisation: 195°C.

<u>Beispiel 4:</u> Zu einer gerührten Lösung, enthaltend 16,5 Teile 3-Oxo-4-(anthrachinon-1-yl)-butyronitril gemäss Beispiel 2 und 100 ml Dimethylformamid, werden 13,5 Teile Natriummethanolat so zugegeben, dass die Temperatur 50°C nicht übersteigt. Man lässt weitere 45 Minuten rühren, kühlt dann die Suspension ab und gibt sie zu einer Lösung aus 50 ml konzentrierter Salzsäure und 600 ml $H_2O$ zu. Der Feststoff wird abgesaugt, gewaschen und getrocknet; es ergeben sich 13 Teile 1H-Anthra[1,9-ef]-benzo[b]furan-2,8-dion der nachstehenden Formel

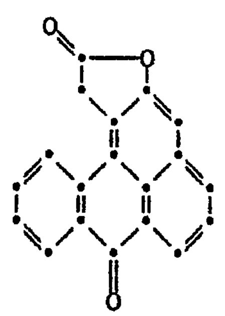

(6)

Schmelzpunkt nach Umkristallisation: 300°C (Zersetzung).

Beispiel 5: Eine wässrige Lösung enthaltend 1,7 Teile 4-Methoxyphenyl-diazoniumchlorid wird zu einer Aufschlämmung aus 2 Teilen 1H-Anthra[1,9-ef]-benzo[b]furan-2,8-dion in 100 ml Dimethylformamid gegeben und das Ganze 24 Stunden lang gerührt. Danach wird der Feststoff abgesaugt und mit Dimethylformamid und Methanol gewaschen. Ausbeute 2,7 Teile 1-(4-Methoxyphenylhydrazono)-anthra[1,9-ef]-benzo[b]furan-2,8-dion der nachstehenden Formel

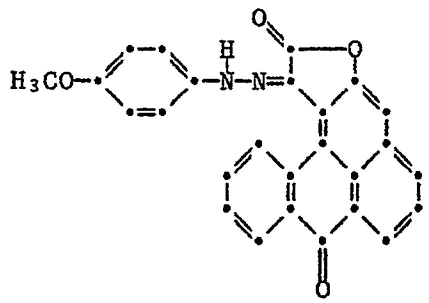

(7),

womit sich Polyesterfasern in einer roten Nuance mit guten Echtheiten färben lassen.

Beispiele 6-10: Arbeitet man wie im Beispiel 5 beschrieben und verwendet statt 4-Methoxyphenyldiazoniumchlorid Diazoniumsalze, die sich von den in der Tabelle genannten Aminen ableiten, so erhält man analoge Farbstoffe, welche Polyesterfasern in roten bis orangen Nuancen anfärben:

| Beispiel | Diazokomponente | $\lambda_{max}$ [nm] |
|---|---|---|
| 6 | Anilin | 482 |
| 7 | 4-Chloranilin | 482 |
| 8 | 4-Nitroanilin | 504 |
| 9 | 3,5-Dinitroanilin | 461 |
| 10 | Sulfanilsäure | 493 |

**Patentansprüche**

1. Benzanthronlactone der Formel

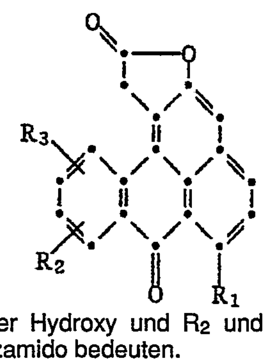

(1) ,

worin $R_1$ Wasserstoff oder Hydroxy und $R_2$ und $R_3$ unabhängig voneinander Wasserstoff, Chlor, Hydroxy, Methoxy oder Benzamido bedeuten.

2. Benzanthronlactone gemäss Anspruch 1, dadurch gekennzeichnet, dass $R_1$ Wasserstoff ist.

3. Benzanthronlactone gemäss einem der Ansprüche 1 oder 2, dadurch gekennzeichnet, dass $R_2$ und $R_3$ unabhängig voneinander Wasserstoff, Chlor oder Benzamido bedeuten.

4. Benzanthronlactone gemäss einem der Ansprüche 1 bis 3, dadurch gekennzeichnet, dass $R_2$ und $R_3$ Wasserstoff sind.

5. Benzanthronlacton gemäss Anspruch 1, dadurch gekennzeichnet, dass $R_1$, $R_2$ und $R_3$ jeweils Wasserstoff sind.

6. Verfahren zur Herstellung von Benzanthronlactonen der Formel (1) gemäss Anspruch 1, dadurch gekennzeichnet, dass man ein Anthrachinon-1-diazoniumsalz der Formel

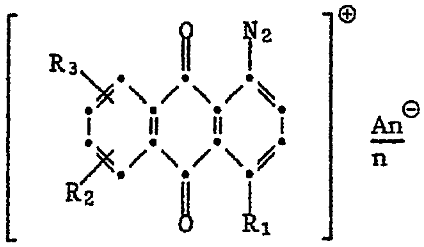 (2) ,

worin $R_1$, $R_2$ und $R_3$ die im Anspruch 1 angegebene Bedeutung haben, An ein Anion ist und n dessen Wertigkeit angibt, mit 2-Methylenglutarsäuredinitril in Gegenwart von katalytischen Mengen eines Metalls der VIII. oder I. Nebengruppe, einem Salz dieser Metalle oder einer Mischung aus einem entsprechenden Metallsalz und Metallpulver in einem organischen Lösungsmittel umsetzt, das hierbei erhältliche Zwischenprodukt der Formel

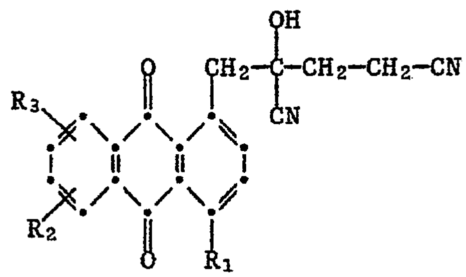 (3)

zum Produkt der Formel

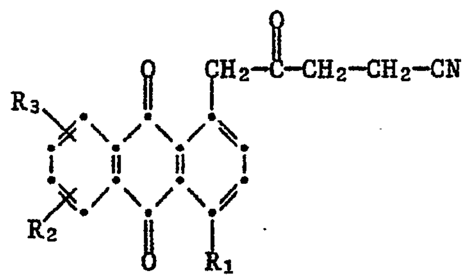 (4)

reagieren lässt und dieses in alkalischem Medium zum Benzanthronlacton der Formel (1) cyclisiert.

7. Verfahren gemäss Anspruch 6, dadurch gekennzeichnet, dass das eingesetzte Anthrachinon-1-diazoniumsalz ein Anthrachinon-1-diazoniumsulfat ist.

8. Verfahren gemäss einem der Ansprüche 6 oder 7, dadurch gekennzeichnet, dass man 1 bis 5 Gew.-%, bezogen auf das Anthrachinonl-diazoniumsalz, Kupfer-(I)-chlorid als Katalysator benutzt.

9. Verfahren gemäss einem der Ansprüche 6 bis 8, dadurch gekennzeichnet, dass das Medium bei der Umsetzung der Verbindung der Formel (3) zur Verbindung der Formel (4) aus einem inerten Lösungsmittel und einem sauren Ionenaustauscher besteht.

10. Verfahren gemäss einem der Ansprüche 6 bis 9, dadurch gekennzeichnet, dass man die Cyclisierung zum Benzanthronlacton in Gegenwart eines Alkalimetallhydroxids, Alkoholats oder Alkalimetallcarbonats in Dimethylformamid, Dimethylsulfoxid, Hexamethylphosphorsäuretriamid oder in einem Glycolether ausführt.

11. Verbindungen der Formel (3) gemäss Anspruch 6, worin $R_1$, $R_2$ und $R_3$ die im Anspruch 1 angegebene Bedeutung haben.

12. Verbindungen der Formel

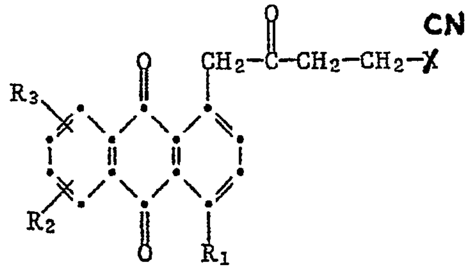

(4$\not{1}$) ,

worin $R_1$, $R_2$ und $R_3$ die im Anspruch 1 angegebene Bedeutung haben.

13. Verbindung gemäss Anspruch 12, dadurch gekennzeichnet, dass $R_1$, $R_2$ und $R_3$ jeweils für Wasserstoff stehen.

14. Verwendung von Benzanthronlactonen gemäss Formel (1) als Kupplungskomponente für die Synthese von Farbstoffen.

15. Farbstoffe der Formel

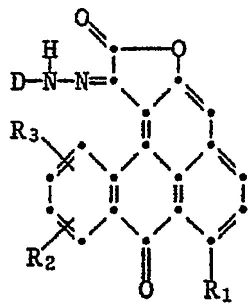

(5) ,

und tautomere Formen davon, worin $R_1$, $R_2$ und $R_3$ die im Anspruch 1 angegebene Bedeutung haben und D der Rest einer Diazokomponente ist.

16. Farbstoffe gemäss Anspruch 15, dadurch gekennzeichnet, dass sich der Diazorest D von einem aromatischen carbocyclischen oder heterocyclischen Amin der allgemeinen Formel

$D-NH_2$

ableitet.

17. Farbstoffe gemäss Anspruch 16, dadurch gekennzeichnet, dass das Amin ein gegebenenfalls weitersubstituiertes Aminobenzol oder eine mono- oder bicyclische Verbindung, die einen aromatisch-heterocyclischen 5- oder 6-Ring aufweist, ist.

18. Farbstoffe gemäss einem der Ansprüche 16 oder 17, dadurch gekennzeichnet, dass das Aminobenzol oder das heterocyclische Amin unsubstituiert oder einfach oder mehrfach mit $C_1$-$C_4$-Alkyl-, $C_1$-$C_4$-Alkoxy-, Halogen-, Nitro-, Cyano-, Sulfo-, Carboalkoxy-, Alkylsulfonyl und/oder Sulfonamid-Resten substituiert ist.

19. Farbstoffe gemäss einem der Ansprüche 15 bis 18, dadurch gekennzeichnet, dass D den Rest eines unsubstituierten oder mit Methoxy, Chlor, Nitro, Cyano und/oder Methyl weitersubstituierten Aminobenzols darstellt.

20. Verwendung von Farbstoffen der Formel (5) gemäss Anspruch 15 zum Färben von synthetischen Fasermaterialien, insbesondere von Polyester-, Polyamid oder Polyolefinfasern.

## Claims

1. A benzanthrone lactone of the formula

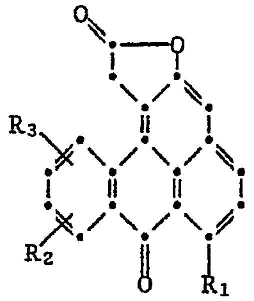

(1) ,

in which $R_1$ is hydrogen or hydroxyl and $R_2$ and $R_3$ independently of one another are hydrogen, chlorine, hydroxyl, methoxy or benzamido.

2. A benzanthrone lactone according to claim 1, in which $R_1$ is hydrogen.

3. A benzanthrone lactone according to either claim 1 or claim 2, in which $R_2$ and $R_3$ independently of one another are hydrogen, chlorine or benzamido.

4. A benzanthrone lactone according to any one of claims 1 to 3, in which $R_2$ and $R_3$ are hydrogen.

5. A benzanthrone lactone according to claim 1, in which $R_1$, $R_2$ and $R_3$ are each hydrogen.

6. A process for the preparation of a benzanthrone lactone of the formula (1) according to claim 1, which comprises reacting a 1-anthraquinone diazonium salt of the formula

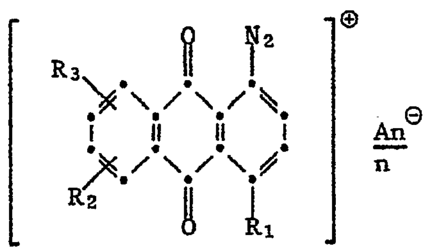

(2) ,

in which $R_1$, $R_2$ and $R_3$ are as defined in claim 1, An is an anion and n is the valency of said anion, with 2-methyleneglutarodinitrile in the presence of a catalytic amount of a metal of auxiliary group VIII or I, of a salt of said metals or of a mixture of a corresponding metal salt and metal powder, in an organic solvent, reacting the intermediate so obtained of the formula

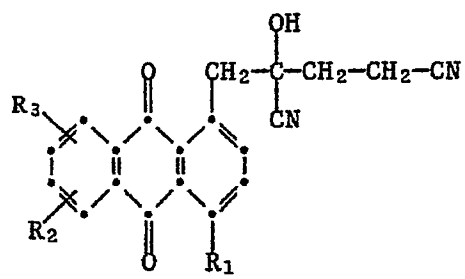

(3)

to form the compound of the formula

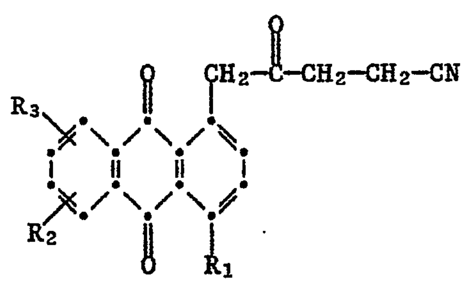

(4)

which is then cyclised, in alkaline medium, to the benzanthrone lactone of the formula (1).

7. A process according to claim 6, wherein the 1-anthraquinone diazonium salt used is a 1-anthraquinone diazonium sulfate.

8. A process according to either claim 6 or claim 7, wherein 1 to 5% by weight, based on the 1-anthraquinone diazonium salt, of cuprous chloride is used as catalyst.

9. A process according to any one of claims 6 to 8, wherein the medium for the reaction of the compound of the formula (3) to the compound of the formula (4) comprises an inert solvent and an acid ion exchanger.

10. A process according to any one of claims 6 to 9, wherein the cyclisation to the benzanthrone lactone is carried out in the presence of an alkali metal hydroxide, an alcoholate or an alkali metal carbonate in dimethylformamide, dimethyl sulfoxide, hexamethylphosphoric triamide or in a glycol ether.

11. A compound of the formula (3) according to claim 6, in which $R_1$, $R_2$ and $R_3$ are as defined in claim 1.

12. A compound of the formula

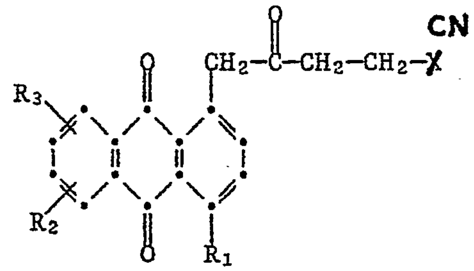

(4) ,

in which $R_1$, $R_2$ and $R_3$ are as defined in claim 1.

13. A compound according to claim 12, wherein $R_1$, $R_2$ and $R_3$ are each hydrogen.

14. Use of a benzanthrone lactone of the formula (1) as coupling component for the synthesis of dyes.

15. A dye of the formula

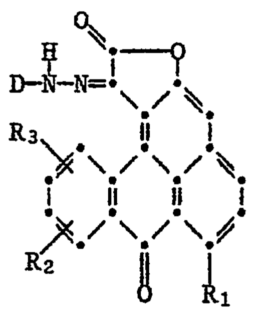

(5) ,

and a tautomer thereof, in which $R_1$, $R_2$ and $R^3$ are as defined in claim 1 and D is the radical of a diazo component.

16. A dye according to claim 15, wherein the diazo radical D is derived from an aromatic carbocyclic or heterocyclic amine of the general formula
$D{-}NH_2$.

17. A dye according to claim 16, wherein the amine is an unsubstituted or further substituted aminobenzene or a mono- or bicyclic compound which contains an aromatic-heterocyclic 5– or 6-membered ring.

18. A dye according to either claim 16 or claim 17, wherein the aminobenzene or the heterocyclic amine is unsubstituted or substituted by one or more radicals of the group $C_1$–$C_4$alkyl, $C_1$–$C_4$alkoxy halogen, nitro, cyano, sulfo, carbalkoxy, alkylsulfonyl and/or sulfonamide.

19. A dye according to any one of claims 15 to 18, wherein D is the radical of an aminobenzene which is unsubstituted or further substituted by methoxy, chlorine, nitro, cyano and/or methyl.

20. Use of a dye of the formula (5) according to claim 15 for dyeing synthetic fibre materials, especially polyester, polyamide or polyolefin fibres.

## Revendications

1. Benzanthrones-lactones de formule

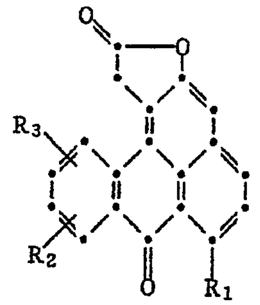

(1) ,

dans laquelle $R_1$ représente l'hydrogène ou un groupe hydroxy et $R_2$ et $R_3$ représentent chacun, indépendamment l'un de l'autre, l'hydrogène, le chlore, un groupe hydroxy, méthoxy ou benzamido.

2. Benzanthrones-lactones selon la revendication 1, caractérisées en ce que $R_1$ représente l'hydrogène.

3. Benzanthrones-lactones selon l'une des revendications 1 ou 2, caractérisées en ce que $R_2$ et $R_3$ représentent chacun, indépendamment l'un de l'autre, l'hydrogène, le chlore ou un groupe benzamido.

4. Benzanthrones-lactones selon l'une des revendications 1 à 3, caractérisées en ce que $R_2$ et $R_3$ représentent l'hydrogène.

5. Benzanthrones-lactones selon la revendication 1, caractérisées en ce que $R_1$, $R_2$ st $R_3$ représentent chacun un atome d'hydrogène.

6. Procédé de préparation des benzanthrones-lactones de formule (1) selon la revendication 1, caractérisé en ce que l'on fait réagir un sel d'anthraquinone-1-diazonium de formule

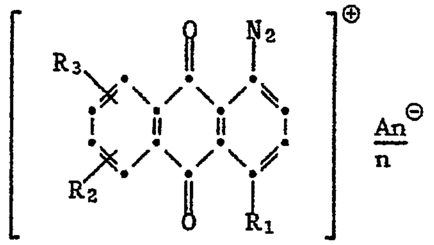

(2) ,

dans laquelle $R_1$, $R_2$ et $R_3$ ont les significations indiquées dans la revendication 1, An représente un anion et n est la valence de ce dernier, avec le 2-méthylène-glutarodinitrile en présence de quantités catalytiques d'un métal du groupe VIII ou d'un sous-groupe I, d'un sel de ces métaux ou d'un mélange d'un sel métallique correspondant et d'une poudre de métal dans un solvant organique, ce qui donne le produit intermédiaire de formule

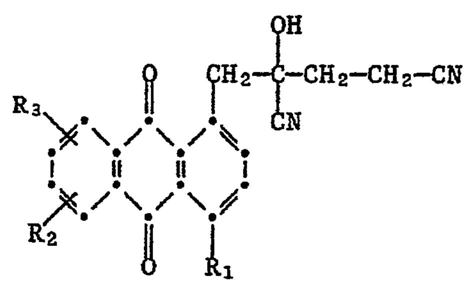

(3)

qu'on convertit en le composé de formule

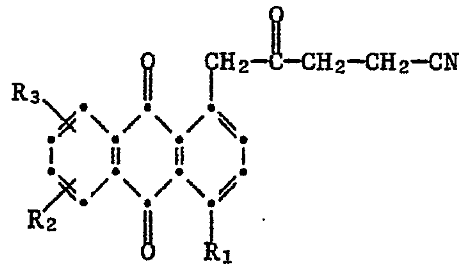

(4)

qu'on convertit en la benzanthrone-lactone de formule (I) par cyclisation en milieu alcalin.

7. Procédé selon la revendication 6, caractérisé en ce que le sel d'anthraquinone-1-diazonium mis en œuvre est un sulfate d'anthraquinone-1-diazonium.

8. Procédé selon l'une des revendications 6 ou 7, caractérisé en ce que l'on utilise en tant que catalyseur le chlorure cuivreux en quantité de 1 à 5% du poids du sel d'anthraquinone-1-diazonium.

9. Procédé selon l'une des revendications 6 à 8, caractérisé en ce que le milieu dans lequel le composé de formule (3) est converti en le composé de formule (4) est constitué d'un solvant inerte et d'un échangeur d'ions acide.

10. Procédé selon l'une des revendications 6 à 9, caractérisé en ce que la cyclisation conduisant à la benzanthrone-lactone est effectuée en présence d'un hydroxyde de métal alcalin, d'un alcoolate ou d'un carbonate de métal alcalin, dans le diméthylformamide, le diméthylsulfoxyde, l'hexaméthylphosphotriamide ou dans un éther de glycol.

11. Composés de formule (3) selon la revendication 6, dans lesquels $R_1$, $R_2$ et $R_3$ ont les significations indiquées dans la revendication 1.

12. Composés de formule

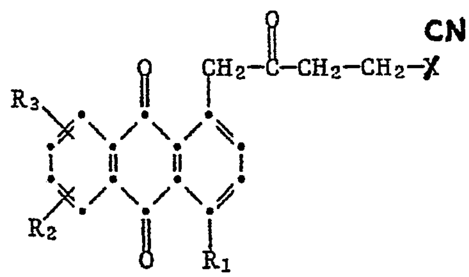

(4), 

dans laquelle $R_1$, $R_2$ et $R_3$ ont les significations indiquées dans la revendication 1.

13. Composés selon la revendication 12, caractérisés en ce que $R_1$, $R_2$ et $R_3$ représentent chacun un atome d'hydrogène.

14. Utilisation des benzanthrones-lactones de formule (1) en tant que copulants dans la synthèse de colorants.

15. Colorants de formule

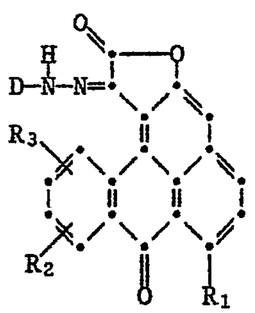

(5) ,

et leurs formes tautomères, dans lesquels $R_1$, $R_2$ et $R_3$ ont les significations indiquées dans la revendication, 1 et D représente le radical d'un composant diazotable.

16. Colorants selon la revendication 15, caractérisés en ce que le radical d'un composant diazotable D dérive d'une amine aromatique carbocyclique ou hétérocyclique de formule générale

$D{-}NH_2$

17. Colorants selon la revendication 16, caractérisés en ce que l'amine est un aminobenzène portant éventuellement d'autres substituants, ou un composé mono- ou bicyclique contenant un cycle aromatique-hétérocyclique à 5 ou 6 chaînons.

18. Colorants selon l'une des revendications 16 ou 17, caractérisés en ce que l'aminobenzène ou l'amine hétérocyclique est non substitué ou ports un ou plusieurs substituants alkyle en $C_1$flC$_4$, alcoxy en $C_1$-$C_4$, halogéno, nitro, cyano, sulfo, carbalcoxy, alkylsulfonyle et/ou sulfonamido.

19. Colorants selon l'une des revendications 15 à 18, caractérisés en ce que D est le radical d'un aminobenzène non substitué ou portant en outre des substituants méthoxy, chloro, nitro, cyano et/ou méthyle.

20. Utilisation des colorants de formule (5) selon la revendication 15, pour la teinture de matières-fibreuses synthétiques, en particulier de fibres de polyesters, de polyamides ou de polyoléfines.